# EUROPEAN PATENT SPECIFICATION

(11) **EP 1 737 504 B1**
(45) Date of publication and mention of the grant of the patent: **09.06.2010**
(21) Application number: 05726039.0
(22) Date of filing: 18.03.2005
(51) Int. Cl.: A61L 24/04, A61L 15/58, C09J 183/04, C08L 83/04

(54) **SILICONE SKIN ADHESIVE GELS**
SILIKON-HAUTKLEBEGELS
GELS ADHESIFS CUTANES EN SILICONE

(30) Priority: 08.04.2004 US 560805 P
(43) Date of publication of application: 03.01.2007
(73) Proprietor: Dow Corning Corporation, Midland, MI 48686-0994 (US)
(72) Inventor: GANTNER, David, Clayton, Midland, MI (US); THOMAS, Xavier, Jean-Paul, B-1640 Rhode-Saint-Genese (BE)
(74) Representative: Russell, Lindsey
(86) International application number: PCT/US2005/009518
(87) International publication number: WO 2005/102403

(56) References cited:
- EP-A- 0 933 410
- EP-A- 0 955 347
- EP-A- 1 083 204
- EP-A- 1 264 864
- WO-A1-2005/051442
- US-A- 4 621 029

## Description

### BACKGROUND OF THE INVENTION

The present invention relates to a method for improving the adhesion of silicone gels to medical substrates by including a hydroxy-substituted siloxane resin in the gel formulations.

Pressure sensitive adhesives (PSAs), including silicone PSAs, and tapes containing such adhesives are known in the art and many are commercially available. Typically, silicone PSAs comprise condensed blends of silicone fluids and silicone resins. Typically, such silicone PSAs are applied as thin coatings between substrates to be adhered together.

It is likewise known in the art to use silicone PSAs in medical applications. For instance, it is known to use silicone PSAs to adhere transdermal drug delivery devices and medical prosthesis to patients.

Silicone PSAs, however, can have a number of properties that limit their use in medical applications. For instance, the adhesive strength of silicone PSAs is often so great that a patient's skin or the object to be adhered can be damaged on removal of the PSA. Additionally, silicone PSAs often exhibit cold flow properties at skin temperature. As such, the resultant inflexible layers of PSA can be very uncomfortable on the patient's body. Moreover, silicone PSAs are harder and more rigid than silicone gels. As such, if the material is to be applied to soft body tissues such as skin, very thin layers must be used to retain the flexibility of the tissue. Finally, silicone PSAs often delaminate from the carrier leaving a coating of the PSA on the skin and/or the object to be adhered. Not only is this a cosmetic problem, but it also limits the ability to reuse the adhesive.

Silicone gels are also known in the art. These gels have been used, for example, as dielectrics, vibration dampers and in medical therapy for cutaneous scars or injuries (e.g., abrasions, surgical areas or bums). In this latter use, the silicone gel is in the form of a sheet with one tacky surface for adherence to the patient's skin and one non-tacky surface to inhibit undesirable adhesion to the gel (e.g., the patient's clothing). Since the silicone gels are softer than PSAs, when they are applied to tissue such as the skin they have a skin-like texture that provides a less disruptive interface with the skin (e.g., it is more flexible).

When silicone gels are joined with certain substrates such as plastics, however, the adhesive strength between the silicone gel and the plastic is often so weak that it delaminates. As such, when the silicone gel is used as an adhesive to adhere a medical substrate to a human or animal body, the gel often delaminates from the substrate before the gel delaminates from the human or animal body to which it is applied.

To solve this problem, the art has suggested treating the substrate such as a plastic surface with means such as corona, flame, and plasma. While such treatments are beneficial, they are difficult to implement on a continuous coating line. Moreover, the treatments must be precisely controlled to prevent damage to the substrate (e.g., heat damage) or inhibit the cure of the silicone material. Finally, such processes often cannot be used on fabric or porous non-woven materials because the uncured gel material can soak through.

A second approach to improve adhesion to substrates, especially plastic substrates, is to use a primer material, such as a titanate material, on the substrate. This approach, however, requires additional processing steps.

The present inventors have now improved the adhesion of silicone gels to medical substrate by including a hydroxy-substituted siloxane resin in the gel formulation.

### SUMMARY OF THE INVENTION

Accordingly, in one of its aspects the present invention provides a silicone gel containing between 2 and 45 wt. % of at least one hydroxy substituted siloxane resin comprising R₃SiO_{1/2} units and SiO_{4/2} units wherein each R is independently a linear, branched or cyclic hydrocarbon group having 1 to 20 carbon atoms and wherein the molar ratio of R₃SiO_{1/2} to SiO_{4/2} is from 0.6:1 to 4:1, for use in adhering a medical substrate to a human or animal comprising affixing a layer of the silicone gel between a medical substrate and a human or animal.

In another of its aspects, the present invention provides a silicone gel comprising the reaction product of 10% to 90 wt.% of an alkenyl-substituted polydiorganosiloxane; 2% to 45 wt.% of a hydroxy functional siloxane resin comprising R₃SiO_{1/2} units and SiO_{4/2} units wherein each R is independently a linear, branched or cyclic hydrocarbon group having 1 to 20 carbon atoms and wherein the molar ratio of R₃SiO_{1/2} to SiO_{4/2} is from 0.6:1 to 4:1; 1% to 30% wt. of an organosiloxane containing silicon-bonded hydrogen atoms; and a hydrosilylation catalyst.

This process can be used to improve the adhesion of the silicone gel to medical substrates such as plastics, natural macromolecular materials (e.g. collagen, wood, cork, leather), metals, glass, ceramics or composites.

The adhesion and physical properties of the gels used in the present invention can be tailored to specific end uses by modifying the gels. Moreover, because of the ease in removability and because the gel generally maintains its tack after removal, the devices of the invention can be reused. In addition, the reusability allows for easy and comfortable repositioning of medical prosthesis. Finally, silicone gels lack cold flow and, as such, are sufficiently soft to allow comfortable use by a human or animal.

### DETAILED DESCRIPTION OF THE INVENTION

The method of the present invention comprises using a hydroxy-substituted siloxane resin in a silicone gel to increase the adhesion of the gel to a medical substrate. The substrate can comprise plastics, natural macromolecular materials (e.g. collagen, wood, cork, leather), metals, glass, ceramics or composites.

The substrates used herein can comprise plastics or resins known in the art. Representative examples include polyolefins including polyethylenes (low density polyethylenes, high density polyethylenes and the like), polypropylenes, polybutylenes, polymethylpentenes, polyethylene-vinyl acetate (EVA) and their copolymers, polyvinyls, polyvinyl acetates, polyvinyl alcohol, polyvinylbutyral, polyvinyl formal, polyurethanes and polyurethane-ureas, polyvinyl chloride derivatives (polyvinyl chloride, polyvinylene chloride, copolyvinylchloride-propylene), polystyrenes and their copolymers (copolystyrene-butadiene, polystyrene acrylonitrile, polyacrylonitrile-butadiene-styrene), polyacrylic and polyacrylates derivatives (polymethyl methacrylate, ethylene/butyl acrylate copolymer, ethylene/methyl acrylate copolymer, ethylene/methacrylic acid copolymer), polyacrylonitrile, polyesters (including PETE, polyethylene terephtalate, polybutylene terephtalate, polyvinylacetate, polylactic-glycolic derivatives), cellulosic films (nitrocellulose, ethylcellulose, cellulose acetate, cellulose acetate butyrate, cellulose propionate), polyimides, polyamides (nylon), epoxy and phenolic plastics, silicone elastomers, polycarbonates, phenoplastes, fluorinated polymers (polytetrafluoroethylene, polyvinylidene fluoride), polyoxymethylenes, polyphenylene oxides, polysulfones (PSU, PESU, PPSU), polyphenyl sulfide, silicones and polysaccharide based materials.

The substrates used in the present invention can also be a continuous or perforated plastic film, a nonwoven film, a knitted fabric, a fiber network, a foam, a metal, a glass or ceramic material.

If desired, the materials can be treated with, for example, a corona, flame, plasma or a primer such as a titanate to increase the adhesion.

In one embodiment, the substrate used is plastic and, alternatively, the plastic is polyurethane.

While the substrate can be in nearly any configuration, in one embodiment of the invention the substrate is in the form of a medical substrate.

As used herein, a "medical substrate" is a substrate that is used in the treatment, maintenance or improvement of a human or animal. Such substrates can be used in combination with other materials for such purpose.

As used herein, a "silicone gel" is an elastic, jelly-like solid material formed by lightly cross-linking silicone polymers. By contrast, a "PSA" has low or no resiliency.

The silicone gel used in the present invention should be chosen to have the properties desired for the end application. Important properties can include softness, friability and strength.

The gels used in the present invention are generally formed from linear or branched silicones having reactive groups thereon. Such reactive groups undergo a cross-linking reaction during curing. Examples of cross-linking reactions include the hydrosilylation reaction in which a silicone having an Si-H reactive group reacts with a silicone having an aliphatically unsaturated reactive group in the presence of a hydrosilylation catalyst. These materials are described, for example in US 5,656,279, US 5,891,076, EP0322118 and US 4,991,574. An alternative reaction is the condensation cure in which a alkoxy and/or hydroxy containing siloxanes are cured with a catalyst as described in US 4,831,070.

In one embodiment, the gels are obtained by reacting at least one alkenyl-substituted polydiorganosiloxane, such as a polydimethylsiloxane having silicon-bonded alkenyl groups such as vinyl, allyl or hexenyl groups, at least one organosiloxane containing silicon-bonded hydrogen atoms, at least one hydroxy containing siloxane resin and at least one catalyst for the reaction of the SiH groups with the Si-alkenyl groups, such as a platinum metal or compounds or complexes thereof. Such compositions cure at normal ambient temperatures, but curing can be expedited by exposure to elevated temperatures, e.g., from about 40°C to about 140°C.

The alkenyl-substituted polydiorganosiloxanes are known in the art as described, for example, in US patent number 3,983,298. Suitable alkenyl groups contain from 2 to about 6 carbon atoms and are exemplified by, but not limited to vinyl, allyl, and hexenyL The alkenyl groups in this component may be located at terminal, pendant (non-terminal), or both terminal and pendant positions. The remaining silicon-bonded organic groups in the alkenyl-substituted polydiorganosiloxane are independently selected from the group consisting of monovalent hydrocarbon and monovalent halogenated hydrocarbon groups free of aliphatic unsaturation. These groups typically contain from 1 to about 20 carbon atoms, alternatively from 1 to 8 carbon atoms and are exemplified by, but not limited, to alkyl such as methyl, ethyl, propyl, and butyl; aryl such as phenyl; and halogenated alkyl such as 3,3,3-trifluoropropyl. In one embodiment of the invention, at least 50 percent of the organic groups in the alkenyl-substituted polydiorganosiloxane are methyl.

The structure of the alkenyl-substituted polydiorganosiloxane is typically linear, however it may contain some branching due to the presence of trifunctional siloxane units. The viscosity of the alkenyl-substituted polydiorganosiloxane can be any desired. For example, it can be less than 100,000 mm²/second, alternatively less than 80,000 mm²/second, and alternatively 300 - 3,000 mm²/second. It should be noted that additional alkenyl-substitution can be included in the form of a resin as described below.

Methods for preparing the alkenyl-substituted polydiorganosiloxanes of the present invention, such as condensation of the corresponding halosilanes or equilibration of cyclic polydiorganosiloxanes, are well known in the art.

The alkenyl-substituted polydiorganosiloxanes can be used in the composition of this invention in an amount of 10-90 wt. % based on the weight of the composition, alternatively 40-90 wt. %, and alternatively 50-80 wt.%. In one embodiment, the amount of alkenyl group present in the alkenyl-substituted polydiorganosiloxane is between 0.05% - 1 % wt%, alternatively 0.05 to 1 wt. % vinyl based on the weight of the alkenyl-substituted polydiorganosiloxane.

The organosiloxane containing silicon-bonded hydrogen atoms are also known in the art as described, for example in US patent number 3,983,298. The hydrogen atoms in this component may be located at terminal, pendant (non-terminal), or both terminal and pendant positions. The remaining silicon-bonded organic groups in this component are independently selected from the group consisting of monovalent hydrocarbon and monovalent halogenated hydrocarbon groups free of aliphatic unsaturation. These groups typically contain from 1 to about 20 carbon atoms, alternatively from 1 to 8 carbon atoms, and are exemplified by, but not limited to alkyl such as methyl, ethyl, propyl, and butyl; aryl such as phenyl; and halogenated alkyl such as 3,3,3-trifluoropropyl. In one embodiment of the invention, at least 50 percent of the organic groups in the organosiloxane containing silicon-bonded hydrogen atoms are methyl.

The structure of the organosiloxane containing silicon-bonded hydrogen atoms is typically linear, however it may contain some branching due to the presence of trifunctional siloxane units. The viscosity of the organosiloxane containing silicon-bonded hydrogen atoms can be any desired. For example, it can be less than 100,000 mm²/second and, alternatively, 5 - 500 mm²/second.

The organosiloxanes containing silicon-bonded hydrogen atoms can be used in the composition of this invention in an amount of 1-30 wt. % based on the weight of the composition, alternatively 5-20 wt %, and alternatively 5-15 wt. %. In one embodiment, the amount of hydrogen group present in the organosiloxane containing silicon-bonded hydrogen atoms is between 0.05% -1.44 wt% based on the weight of the organosiloxane containing silicon-bonded hydrogen atoms.

Methods of preparing the organosiloxane containing silicon-bonded hydrogen atoms of the present invention by co-hydrolysis of the appropriate chlorosilanes are known in the art. U.S. Patent No. 2,877,255 to Clark; Japanese Laid Open Patent Application (KOKAI) SHO 62(1987)-39660 to Mogi et al.; and U.S. Patent No.s 5,446,185 and U.S. No. 5,493,040 to Cobb et al.

The ratio of (H as SiH)/(Alkenyl as Si-Alkenyl) is generally in the range of 0.1:1 to 10:1.

The hydrosilylation catalyst promotes the addition reaction of the alkenyl-substituted polydiorganosiloxane with the organosiloxane containing silicon-bonded hydrogen. The hydrosilylation catalyst can be any of the well known hydrosilylation catalysts comprising a platinum group metal, a compound containing a platinum group metal, or a microencapsulated platinum group metal or compound containing same. These platinum group metals include platinum, rhodium, ruthenium, palladium, osmium and iridium. Platinum and platinum compounds are preferred catalysts based on their high activity level in hydrosilylation reactions. One class of platinum catalysts is the complexes of chloroplatinic acid with certain vinyl-containing organosiloxane compounds disclosed by Willig in U.S. Pat. No. 3,419,593. A specific catalyst of this type is the reaction product of chloroplatinic acid and 1,3-diethenyl-1,1,3,3-tetramethyldisiloxane.

The hydrosilylation catalyst is present in an amount sufficient to cure the composition of the present invention. Typically, the concentration of the catalyst is sufficient to provide from 0.1 to 500 ppm (part per million), alternatively from 1 to 100 ppm, alternatively from 1 to 50 parts per million of a platinum group metal, based on the combined weight of the other components.

The gels of the present invention also contain at least one hydroxy-substituted siloxane resin. This resin increases the adhesion of the gel to the medical substrate and the skin. This resin comprises R₃SiO_{1/2} units (M units) and SiO_{4/2} units (Q units) wherein each R is independently a linear, branched or cyclic hydrocarbon group having 1-20 carbon atoms. R can be unsubstituted or substituted with halogen atoms. Each R can be identical or different, as desired. The hydrocarbon group of R can be exemplified by alkyl groups such as methyl, ethyl, propyl, butyl, hexyl, octyl3,3,3-trifluoropropyl, chloromethyl, and decyl, alkenyl groups such as vinyl and hexenyl, cycloaliphatic groups such as cyclohexyl, aryl groups such as phenyl, tolyl, and xylyl, chlorophenyl, and aralkyl groups such as benzyl, styryl and alpha-methylstyryl. Alternatively, each R group is an independently selected alkyl or alkenyl group comprising 1 to 8 carbon atoms or aryl group comprising 6 to 9 carbon atoms. Alternatively, each R group is independently selected from methyl and vinyl.

If an alkenyl group is present in the resin, typically the mole % of R groups present as alkenyl groups is less than 10%, alternatively less than 5%. For example, if the resin contains vinyl groups, typically they are present in an amount of less than 5 wt. % of the resin solids, alternatively less than 2.5 wt. % of the resin solids, alternatively 1.5-2 wt. % of the resin solids

The molar ratio of R₃SiO_{1/2} (M units) to SiO_{4/2} (Q units) is from 0.6:1 to 4:1. Alternatively, the molar ratio of M:Q can be from 0.6:1 to 1.9:1. Alternatively, the molar ratio of M:Q can be from 0.6:1 to 1.0:1. The resins can also contain triorganosiloxy units (T units), for example 0.5 to 1 triorganosiloxy group for every SiO_{4/2} unit, alternatively 0.6 to 0.9 triorganosiloxy group for every SiO_{4/2} unit.

It should be noted that more than 1 resin could be included in the present invention. In this case, at least one of the resins should have the silanol content as described below but, by the same token, one could have the silanol capped so that there is substantially no silanol present. It should also be noted that other resins can also be added to the composition of this invention. For example, organic resins could be added if desired. In one embodiment, for example, a vinyl-functional organic resin can be added.

In one embodiment a majority of all R groups are methyl and the total number of R groups that have olefinic unsaturation is no more than 0.5% of all R groups. In another embodiment substantially all of the R groups are methyl. In another embodiment substantially all of the R groups are substantially free of olefinic unsatuaration. In yet another embodiment of the invention, 2 resins are included - one in which substantially all of the R groups are methyl and the other in which 3.5 to 4 mole % of the R groups are vinyl and substantially all of the remaining R groups are methyl.

The resins also contain silicon-bonded hydroxyl groups ranging from about 0.01 up to 5 weight percent of the resin, alternatively from about 1 to about 5 wt. % of the resin.

Resins comprising R₃SiO_{1/2} units and SiO_{4/2} units are well known in the art. These copolymers are described, for example, in U.S. Pat Nos. 3,936,582, 2,676,182, and 2,857,356.

The resinous copolymers can be prepared by cohydrolysis of a mixture of silanes having four hydrolyzable groups, e.g., silicon tetrachloride, and triorganosilanes having one hydrolyzable group, e.g., trimethylchlorosilane, in the proper ratio. A specific method for the preparation of these resinous copolymers is described in U.S. Pat. No. 2,676,182, wherein a silica hydrosol is reacted under acidic conditions with a source of triorganosiloxy units such as a hexaorganodisiloxane, for example, hexamethyldisiloxane, or a hydrolyzable triorganosilane, for example, trimethylchlorosilane, or mixtures thereof.

The resins can be used in the composition of this invention in an amount of 2-45 wt. % based on the weight of the composition, alternatively 5-40 wt. % and alternatively 10-35 wt. %.

If desired, other components can be included in the gels of the present invention including, but not limited to, fillers, pigments, low temperature cure inhibitors, additives for improving adhesion, cross-linkers (e.g., Si-H cross-linkers), chain extended pharmaceutical agents, cosmetic agents, natural extracts, fluids or other materials conventionally used in gels. Other optional components include silicone fluids, silicone waxes, silicone polyethers, and other polymers including, for example, hydrophilic polymers such as sodium polyacrylic acid, PVA, PVC, polyacrylic adhesive, cellulose and polysaccharide (which can make the gel more hydrophilic and more permeable to moisture). Still other optional components include rheology modifiers such as thickening agents, thixotropic agents and materials that react with the ingredients if the gel such as castor oil or maleates that can react with the hydroxyl groups of the resin. In one embodiment, the gel contains substantially no filler (e.g., less than 5 wt.%,' alternatively less than 1 wt. %, alternatively less than 0.1 wt. %). In another embodiment, the gel contains substantially no solvent (e.g., less than 5 wt.%, alternatively less than 1 wt. %, alternatively less than 0.1 wt. %).

Active agents to be used in this invention can include any solid or liquid material that can be bound in the composition and subsequently released at the desired rate. The active agents should also not interfere with the curing of the silicone formulation to an unacceptable extent. Suitable active agents include cosmetics, personal care, cosmeceuticals, therapeutic or diagnostic materials, pesticides, herbicides, and the like.

Therapeutic active agents which may be employed include, for example, antiacne agent, antibiotic, antiseptic, autifungal, antibacterial, antimicrobial, biocides, antiinflammatory, astringents, hormones, anticancer agents, smoking cessation compositions, cardiovascular, histamine blocker, bronchodilator, analgesic, antiarrythmic, antihistamine, alpha-I blocker, beta blocker, ACE inhibitor, diuretic, antiaggregant, sedative, tranquillizer, anticonvulsant, anticoagulant agents, vitamins, antiaging agents, agents for treating gastric and duodenal ulcers, anticellulites, proteolytic enzymes, healing factors, cell growth nutrients, peptides and others. Specific examples of suitable therapeutic active agents include penicillins, cephalosporins, tetracyclines, macrolides, epinephrine, amphetamines, aspirin, acetominophen, barbiturates, catecholamines, benzodiazepine, thiopental, codeine, morphine, procaine, lidocaine, benzocaine, sulphonamides, ticonazole, perbuterol, furosanoide, prazosin, prostaglandins, salbutamol, indomethicane, diclofenac, glafenine, dipyridamole, theophylline and retinol.

In addition to the therapeutic or diagnostic materials, active, agents could be cosmetics such as perfumes, UV protectors, shaving products, deodorants or the like. Suitable cosmetics are known to those skilled in the art.

Some additional examples of the cosmetics, personal care, and cosmeceutical ingredients and pharmaceutical excipients that may be used herein may be found in the CTFA ingredient Database and the handbook of pharmaceutical excipients and can include, for examples, absorbents, anticacking agents, antioxidants, antistatic agents, astringents, binders, buffering agents, bulking agents, chelating agents, colorants, cosmetic astringents, cosmetic biocides, deodorant agents, emollients, external analgesics, film formers, flavoring agents, fragrance ingredients, humectants, lytic agents, moisturizing agents, occlusivity enhancers, opacifying agents, oxidizing and reducing agents, penetration enhancers, pesticides, plasticizers, preservatives, skin bleaching agents, skin conditioning agents, skin protectants, slip modifiers, solubilizing agents, solvents, sunscreen agents, surface modifiers, surfactants and emulsifying agents, suspending agents, thickening agents, viscosity controlling agents including increasing or decreasing agents, UV light absorbers,

Cosmetic, personal care and cosmeceutical ingredients, and pharmaceutical excipients which may be employed are selected, for example, from the following chemical classes: alcohols, fatty alcohols and polyols, aldehydes, alkanolamines, alkoxylated alcohols (e.g. polyethylene glygol derivatives of alcohols and fatty alcohols), alkoxylated amides, alkoxylated amines, alkoxylated carboxylic acids, amides including salts (e.g. ceramides), amines, amino acids including salts and alkyl substituted derivatives, esters, alkyl substituted and acyl derivatives, polyacrylic acids, acrylamide copolymers, adipic acid copolymers, alcohols, aminosilicones, biological polymers and derivatives, butylene copolymers, carbohydrates (e.g. polysaccharides, chitosan and derivatives), carboxylic acids, carbomers, esters, ethers and polymeric ethers (e.g. PEG derivatives, PPG derivatives), glyceryl esters and derivatives, halogen compounds, heterocyclic compounds including salts, hydrophilic colloids and derivatives including salts and gums (e.g. cellulose derivatives, gelatin, xanthan gum, natural gums), imidazolines, inorganic materials (clay, TiO2, ZnO), ketones (e.g. camphor), isethionates, lanolin and derivatives, organic salts, phenols including salts (e.g. parabens), phosphorus compounds (e.g. phosphate derivatives), polyacrylates and acrylate copolymers, protein and enzymes derivatives (e.g. collagen), synthetic polymers including salts, siloxanes and silanes, sorbitan derivatives, sterols, sulfonic acids and derivatives and waxes.

Some examples of antiacne agents are Salicylic acid and Sulfur. Some examples of antifungal agents are Calcium Undecylenate, Undecylenic Acid, Zinc Undecylenate, and Povidone-Iodine. Some examples of antimicrobial agents are Alcohol, Benzalkonium Chloride, Benzethonium Chloride, Hydrogen Peroxide, Methylbenzethonium Chloride, Phenol, Poloxamer 188, and Povidone-Iodine. Some examples of antioxidants are Acetyl Cysteine, Arbutin, Ascorbic Acid, Ascorbic Acid Polypeptide, Ascorbyl Dipalinitate, Ascorbyl Methylsilanol Pectinate, Ascorbyl Palmitate, Ascorbyl Stearate, BHA, p-Hydroxyanisole, BHT, t-Butyl Hydroquinone, Caffeic Acid, Camellia Sinensis Oil, Chitosan Ascorbate, Chitosan Glycolate, Chitosan Salicylate, Chlorogenic Acids, Cysteine, Cysteine HCI, Decyl Mercaptomethylimidazole, Erythorbic Acid, Diamylhydroquinone, Di-t-Butylhydroquinone, Dicetyl Thiodipropionate, Dicyclopentadiene/t-Butylcresol Copolymer, Digalloyl Trioleate, Dilauryl Thiodipropionate, Dimyristyl Thiodipropionate, Dioleyl Tocopheryl Methylsilanol, Isoquercitrin, Diosmine, Disodium Ascorbyl Sulfate, Disodium Rutinyl Disulfate, Distearyl Thiodipropionate, Ditridecyl Thiodipropionate, Dodecyl Gallate, Ethyl Ferulate, Ferulic Acid, Hydroquinone, Hydroxylamine HCI, Hydroxylamine Sulfate, Isooctyl Thioglycolate, Kojic Acid, Madecassicoside, Magnesium Ascorbate, Magnesium Ascorbyl Phosphate, Melatonin, Methoxy-PEG-7 Rutinyl Succinate, Methylene Di-t-Butylcresol, Methylsilanol Ascorbate, Nordihydroguaiaretic Acid, Octyl Gallate, Phenylthioglycolic Acid, Phloroglucinol, Potassium Ascorbyl Tocopheryl Phosphate, Thiodiglycolamide, Potassium Sulfite, Propyl Gallate, Rosmarinic Acid, Rutin, Sodium Ascorbate, Sodium Ascorbyl/Cholesteryl Phosphate, Sodium Bisulfite, Sodium Erythorbate, Sodium Metabisulfide, Sodium Sulfite, Sodium Thioglycolate, Sorbityl Furfural, Tea Tree (Melaleuca Aftemifolia) Oil, Tocopheryl Acetate, Tetrahexyldecyl Ascorbate, Tetrahydrodiferuloylmethane, Tocopheryl Linoleate/Oleate, Thiodiglycol, Tocopheryl Succinate, Thiodiglycolic Acid, Thioglycolic Acid, Thiolactic Acid, Thiosalicylic Acid, Thiotaurine, Retinol, Tocophereth-5, Tocophereth-10, Tocophereth-12, Tocophereth-18, Tocophereth-50, Tocopherol, Tocophersolan, Tocopheryl Linoleate, Tocopheryl Nicotinate, Tocoquinone, o-Tolyl Biguanide, Tris(Nonylphenyl) Phosphite, Ubiquinone, and Zinc Dibutyldithiocarbamate. Some examples of cosmetic biocides are Aluminum Phenolsulfonate, Ammonium Phenolsulfonate, Bakuchiol, Benzalkonium Bromide, Benzalkonium Cetyl Phosphate, Benzalkonium Chloride, Benzalkonium Saccharinate, Benzethonium Chloride, Potassium Phenoxide, Benzoxiquine, Benzoxonium Chloride, Bispyrithione, Boric Acid, Bromochlorophene, Camphor Benzalkonium Methosulfate, Captan, Cetalkonium Chloride, Cetearalkonium Bromide, Cetethyldimonium Bromide, Cetrimonium Bromide, Cetrimonium Chloride, Cetrimonium Methosulfate, Cetrimonium Saccharinate, Cetrimonium Tosylate, Cetylpyridinium Chloride, Chloramine T, Chlorhexidine, Chlorhexidine Diacetate, Chlorhexidine Digluconate, Chlorhexidine Dihydrochloride, p-Chloro-m-Cresol, Chlorophene, p-Chlorophenol, Chlorothymol, Chloroxylenol, Chlorphenesin, Ciclopirox Olamine, Climbazole, Cloflucarban, Clotrimazole, Coal Tar, Colloidal Sulfur, o-Cynlen-5-ol, Dequalinium Acetate, Dequalinium Chloride, Dibromopropamidine Diisethionate, Dichlorobenzyl Alcohol, Dichlorophene, Dichlorophenyl Imidazoldioxolan, Dichloro-m-Xylenol, Diiodomethyltolylsulfone, Dimethylol Ethylene Thiourea, Diphenylmethyl Piperazinylbenzimidazole, Domiphen Bromide, 7-Ethylbicyclooxazolidine, Fluorosalan, Formaldehyde, Glutaral, Hexachlorophene, Hexamidine, Hexamidine Diisethionate, Hexamidine Diparaben, Hexamidine Paraben, Hexetidine, Hydrogen Peroxide, Hydroxymethyl Dioxoazabicyclooctane, Ichthammol, Isopropyl Cresol, Lapyrium Chloride, Lauralkonium Bromide, Lauralkonium Chloride, Laurtrimonium Bromide, Laurtrimonium Chloride, Laurtrimonium Trichlorophenoxide, Lauryl Isoquinolinium Bromide, Lauryl Isoquinolinium Saccharinate, Laurylpyridinium Chloride, Mercuric Oxide, Methenamine, Methenammonium Chloride, Methylbenzethonium Chloride, Myristalkonium Chloride, Myristalkonium Saccharinate, Myrtrimonium Bromide, Nonoxynol-9 Iodine, Nonoxynol-12 Iodine, Olealkonium Chloride, Oxyquinoline, Oxyquinoline Benzoate, Oxyquinoline Sulfate, PEG-2 Coco-Benzonium Chloride, PEG-10 Coco-Benzonium Chloride, PEG-6 Undecylenate, PEG-8 Undecylenate, Phenol, o-Phenylphenol, Phenyl Salicylate, Piroctone Olamine, Sulfosuccinylundecylenate, Potassium o-Phenylphenate, Potassium Salicylate, Potassium Troclosene, Propionic Acid, PVP-Iodine, Quaternium-8, Quaternium-14, Quaternium-24, Sodium Phenolsulfonate, Sodium Phenoxide, Sodium o-Phenylphenate, Sodium Shale Oil Sulfonate, Sodium Usnate, Thiabendazole, 2,2'-Thiobis(4-Chlorophenol), Thiram, Triacetin, Triclocarban, Triclosan, Trioctyldodecyl Borate, Undecylenamidopropylamine Oxide, Undecyleneth?6, Undecylenic Acid, Zinc Acetate, Zinc Aspartate, Zinc Borate, Zinc Chloride, Zinc Citrate, Zinc Cysteinate, Zinc Dibutyldithiocarbamate, Zinc Gluconate, Zinc Glutamate, Zinc Lactate, Zinc Phenolsulfonate, Zinc Pyrithione, Zinc Sulfate, and Zinc Undecylenate. Some examples of external analgesics are Benzyl Alcohol, Capsicum Oleoresin (Capsicum Frutescens Oleoresin), Methyl Salicylate, Camphor, Phenol, Capsaicin, Juniper Tar (Juniperus Oxycedrus Tar), Phenolate Sodium (Sodium Phenoxide), Capsicum (Capsicum Frutescens), Menthol, Resorcinol, Methyl Nicotinate, and Turpentine Oil (Turpentine). Some examples of oxidizing agents are Ammonium Persulfate, Calcium Peroxide, Hydrogen Peroxide, Magnesium Peroxide, Melamine Peroxide, Potassium Bromate, Potassium Caroate, Potassium Chlorate, Potassium Persulfate, Sodium Bromate, Sodium Carbonate Peroxide, Sodium Chlorate, Sodium Iodate, Sodium Perborate, Sodium Persulfate, Strontium Dioxide, Strontium Peroxide, Urea Peroxide, and Zinc Peroxide. Some examples of reducing agents are Ammonium Bisufite, Ammonium Sulfite, Ammonium Thioglycolate, Ammonium Thiolactate, Cystemaine HCl, Cystein, Cysteine HCl, Ethanolamine Thioglycolate, Glutathione, Glyceryl Thioglycolate, Glyceryl Thioproprionate, Hydroquinone, p-Hydroxyanisole, Isooctyl Thioglycolate, Magnesium Thioglycolate, Mercaptopropionic Acid, Potassium Metabisulfite, Potassium Sulfite, Potassium Thioglycolate, Sodium Bisulfite, Sodium Hydrosulfite, Sodium Hydroxymethane Sulfonate, Sodium Metabisulfite, Sodium Sulfite, Sodium Thioglycolate, Strontium Thioglycolate, Superoxide Dismutase, Thioglycerin, Thioglycolic Acid, Thiolactic Acid, Thiosalicylic Acid, and Zinc Formaldehyde Sulfoxylate. An example of a skin bleaching agent is Hydroquinone. Some examples of skin protectants are Allantoin, Aluminum Acetate, Aluminum Hydroxide, Aluminum Sulfate, Calamine, Cocoa Butter, Cod Liver Oil, Colloidal Oatmeal, Dimethicone, Glycerin, Kaolin, Lanolin, Mineral Oil, Petrolatum, Shark Liver Oil, Sodium Bicarbonate, Talc, Witch Hazel, Zinc Acetate, Zinc Carbonate, and Zinc Oxide. Some examples of sunscreen agents are Aminobenzoic Acid, Cinoxate, Diethanolamine Methoxycinnamate, Digalloyl Trioleate, Dioxybenzone, Ethyl 4-[bis(Hydroxypropyl)] Aminobenzoate, Glyceryl Aminobenzoate, Homosalate, Lawsone with Dihydroxyacetone, Menthyl Anthranilate, Octocrylene, Octyl Methoxycinnamate, Octyl Salicylate, Oxybenzone, Padimate O, Phenylbenzimidazole Sulfonic Acid, Red Petrolatum, Sulisobenzone, Titanium Dioxide, and Trolamine Salicylate. Some examples of UV light absorbing agents are Acetaminosalol, Allatoin PABA, Benzalphthalide, Benzophenone, Benzophenone 1-12, 3-Benzylidene Camphor, Benzylidenecamphor Hydrolyzed Collagen Sulfonamide, Benzylidene Camphor Sulfonic Acid, Benzyl Salicylate, Bomelone, Bumetriozole, Butyl Methoxydibenzoylmethane, Butyl PABA, Ceria/Silica, Ceria/Silica Talc, Cinoxate, DEA-Methoxycinnamate, Dibenzoxazol Naphthalene, Di-t-Butyl Hydroxybenzylidene Camphor, Digalloyl Trioleate, Diisopropyl Methyl Cinnamate, Dimethyl PABA Ethyl Cetearyldimonium Tosylate, Dioctyl Butamido Triazone, Diphenyl Carbomethoxy Acetoxy Naphthopyran, Disodium Bisethylphenyl Tiamminotriazine Stilbenedisulfonate, Disodium Distyrylbiphenyl Triaminotriazine Stilbenedisulfonate, Disodium Distyrylbiphenyl Disulfonate, Drometrizole, Drometrizole Trisiloxane, Ethyl Dihydroxypropyl PABA, Ethyl Diisopropylcinnamate, Ethyl Methoxycinnamate, Ethyl PABA, Ethyl Urocanate, Etrocrylene Ferulic Acid, Glyceryl Octanoate Dimethoxycinnamate, Glyceryl PABA, Glycol Salicylate, Homosalate, Isoamyl p-Methoxycinnamate, Isopropylbenzyl Salicylate, Isopropyl Dibenzolylmethane, Isopropyl Methoxycinnamate, Menthyl Anthranilate, Menthyl Salicylate, 4-Methylbenzylidene, Camphor, Octocrylene, Octrizole, Octyl Dimethyl PABA, Octyl Methoxycinnamate, Octyl Salicylate, Octyl Triazone, PABA, PEG-25 PABA, Pentyl Dimethyl PABA, Phenylbenzimidazole Sulfonic Acid, Polyacrylamidomethyl Benzylidene Camphor, Potassium Methoxycinnamate, Potassium Phenylbenzimidazole Sulfonate, Red Petrolatum, Sodium Phenylbenzimidazole Sulfonate, Sodium Urocanate, TEA-Phenylbenzimidazole Sulfonate, TEA-Salicylate, Terephthalylidene Dicamphor Sulfonic Acid, Titanium Dioxide, TriPABA Panthenol, Urocanic Acid, and VA/Crotonates/Methacryloxybenzophenone-1 Copolymer.

The consistency, strength, softness and tackiness of the gel is determined by a number of factors including the ratio of reactive groups in the materials, the viscosity of the polymers, the quantity of ingredients including resin and the like. In one embodiment of the invention, the composition used in the present invention comprises 65-75 wt. % of an alkenyl-substituted polydiorganosiloxane, 1-3 wt. % of a second alkenyl-substituted polydiorganosiloxane, 3-6 wt. % of a vinyl and hydroxy substituted siloxane resin, 9-15 wt. % of a hydroxy substituted siloxane resin, 9-15 wt. % of a organosiloxane containing terminal silicon-bonded hydrogen atoms and a hydrosilylation catalyst which has relatively low adhesion to the skin. In another embodiment of the invention, the composition used in the present invention comprises 60-70 wt. % of an alkenyl-substituted polydiorganosiloxane, 5-7 wt. % of a vinyl and hydroxyl substituted siloxane resin, 6-10 wt. % of a hydroxy substituted siloxane resin, 8-12 wt. % of an organosiloxane containing terminal silicon-bonded hydrogen atoms and a hydrosilylation catalyst which has relatively medium adhesion to the skin. In another embodiment of the invention, the composition used in the present invention comprises 70-80 wt % of an alkenyl-substituted polydiorganosiloxane, 3-8 wt % of a vinyl and hydroxy substituted siloxane resin, 9-15 wt. % of a hydroxy substituted siloxane resin, 8-12 wt. % of an organosiloxane containing terminal silicon-bonded hydrogen atoms and a hydrosilylation catalyst which has relatively medium-high adhesion to the skin. In another embodiment of the invention, the composition used in the present invention comprises 50-70 wt. % of an alkenyl-substituted polydiorganosiloxane, 12-18 wt. % of a vinyl and hydroxy substituted siloxane resin, 12-18 wt. % of a hydroxy substituted siloxane resin, 6-10 wt. % of an organosiloxane containing terminal silicon-bonded hydrogen atoms and a hydrosilylation catalyst which has relatively high adhesion to the skin. In another embodiment of the invention the composition used in the present invention comprises 25-45 wt. % of an alkenyl-substituted polydiorganosiloxane, 10-20 wt. % of a vinyl and hydroxy substituted siloxane resin, 15-25 wt. % of a hydroxy substituted siloxane resin, 6-10 wt. % of an organosiloxane containing terminal silicon-bonded hydrogen atoms and a hydrosilylation catalyst which has relatively high adhesion to the skin.

As measured by the Cone Penetration Test method based on ASTM D-217-88, gels in one embodiment of the invention have a penetration of 50 mm to 300 mm with a cone category 1806-1 weighted 62.5 g.

Generally, the gels have a coating weight in the range of 100 to 4500 g/m². In one embodiment of the invention, the densities are in the range of 150 to 1200 g/m². Such gels would generally have thicknesses in the range of 0.15 to 5 mm, and in one embodiment of the invention the gels have a thickness of 0.2 to 1.5 mm.

The adhesive strength of the silicone gels should be sufficient to maintain adhesion to the medical substrate. Similarly, if the gel is to be adhered to a human or animal, the adhesive strength of the gel should be sufficient to ensure that it remains attached to the human or animal and yet not so strong that the human or animal is not excessively damaged when the gel is removed. As noted above, traditional silicone gels often do not adhere well to certain substrates such as plastics and, thus, delaminate. As such, the applicants herein have discovered that by adding a hydroxy-substituted siloxane resin to the silicone gel, the adhesive strength of the silicone gel to the plastic substrate is increased. When measured with a Probe Tack Tester, the tack is generally between 50 g and 500 g, and in one embodiment of the invention the tack is in the range of 150 g to 350 g. The adhesive property can also be measured using a Texture Analyzer (1/2 inch diameter cylinder Derlin probe, 100 gf applied for 5 seconds and 10 mm/s separation speed) the tack is generally between 50 gf to 500 gf.

When used on a human or animal, the silicone gels should also be sufficiently soft and flexible to ensure comfort to the user. However, since softness also generally results in weaker gels, these two factors should be considered in selection and formulation of the gel.

If desired, the surfaces of the gels to be adhered to the medical substrate or the patient can be covered or protected with a release liner prior to use. The adhesive strength between the silicone gel and such release liner is obviously less than that between the gel and the substrate such that the release liner can be peeled off of the silicone gel revealing the underlying tacky gel. Suitable release liner materials are known in the art and can include, for instance, a plastic or multi-ply material such as a silicone, a fluorinated silicone, a fluorine polymer, polyethylene, polypropylene, polyethylene terephthalate (PET), ethylvinyl acetate polymer, a PVC or the like. Additionally, the release liner could be made from a wide variety of materials (e.g., paper) coated with a suitable release coating. Finally, the surface of the release coating can be smooth, embossed or in any other desirable form.

If the adhesive strength of the gel is still not sufficient to adhere it to the medical substrate, either the surface of the gel or the surface of the plastic can be primed. Suitable primers include titanate materials such as organic titanates commercialized by Dupont® under Tradename Tyzor®; organic zirconate; hydrogen functional siloxanes such as dimethyl, methylhydrogen siloxane, trimethylsiloxy-terminated, methylhydrogen siloxane, trimethylsiloxy-terminated, dimethyl siloxane, hydrogen-terminated, and methylhydrogen cyclosiloxanes; and platinum derivatives such as 1,3-diethenyl-1,1,3,3 -tetramethyldisiloxane complexes (platinum).

The composition of the present invention can be made by mixing the components in the desired ratio. This can be done in conventional mixing equipment such as static mixers and the like. Typically, the order of mixing is not important other than curing may initiate when the Si-H, Si-Vinyl and hydrosilylation catalyst are mixed. If desired, the composition can be pre-compounded and supplied in multi-part system, preferably as a two-part A & B system.

The gel layer can also be made by any desirable technique. One example comprises preforming the gel (e.g., as a sheet) by known procedures e.g. by molding, calendering, extruding, spraying, brushing, applying by hand, coating or casting on, for example, a releasable substrate. The gel is then brought together with the medical substrate. Alternatively, the gel may be preformed (e.g., as a sheet) by casting and curing the gel-forming composition on the medical substrate.

In the above processes, the gel forming composition may be applied by techniques such as spraying, coating, bar coating, etc. If desired, the gel forming composition can be used as a dispersion or solution in a volatile solvent such as an organic solvent, a low molecular weight silicone or other suitable solvent and, thereafter, the solvent can be evaporated. An alternative method is to use a gel forming composition that does not require a substantial quantity of solvent, for example less than 5 wt. %, alternatively less than 1 wt. %.

In the above process, the gel forming composition may be applied as a continuous layer, a coating with void areas, a perforated layer, a patterned layer, or a discontinuous layer forming various designs such as lines, dots, circles, networks etc.

The substrate onto which the gel is applied in the above processes can be any surface that will impart the desired configuration to the compositions. Thus, it may be a continuous belt onto which the gel forming composition is spread. Depending on the consistency of the compositions, the substrate may have barriers at its edges to restrict the flow of the compositions until cure takes place. In one embodiment of the invention, the substrate is a releaseable substrate to allow the gel to be easily removed and used.

If desired, the substrate can be a preformed blister package made of any of the conventional blister packaging materials including, for example, polyvinyl chloride, polypropylene, polyethylene, polyester, paper or composites with or without suitable release coatings.

The composition formed above can be any size and shape desired based on the final use. For instance, it can be circular, square or rectangular and it can vary from a few square centimeters to in excess of several hundred square cm.

The compositions of the present invention are useful in applications where the adhesion provided by a silicone gel is useful. The silicone gel adhesives of this invention are, however, particularly adapted for adhering medical substrates on patients. Examples of such substrates include devices such as breast prosthesis, catheters, cannulas, drainage bags, uridomes, incontinence devices, hygiene napkins, pouches, false hairpieces (e.g., toupees), tubes, ostomy and related devices, surgery drapes, facial masks, gloves, other medical devices and the like. For example, these materials can be used for the manufacturing of silicone adhesive tapes (e.g. PU non-woven fabric with gel on it), gel sheeting (e.g. PU film with gel on it), wound dressings (e.g. PU film or PU foam with gel on it), bandages, adhesive strips, surgery drapes (PE film with gel on it), adhesive medical devices (e.g. PVC tube with gel, latex catheter with gel on it), hygiene napkins (PE film with gel), external prosthesis (e.g. PU external breast prosthesis with gel on it), topical or transdermal patches (e.g., drug delivery), fragrance/cosmetics patches, and the like.

The following Examples illustrate the invention. Unless otherwise indicated, all percentages are by weight and all viscosities are at 25°C.

### Sample preparation and testing

- The viscosity of the materials before cure is measured using a Brookfield viscosimeter
- The softness of the gels in mm is measured using a PNRG penetrometer with a 65mm diameter/62.5g cone probe. The sample tested is prepared by mixing the ingredients, pouring 80g to 100g of the sample in a plastic cup and curing it at 103°C-140°C for 60 min.
- The tack in gf (gramforce) is measured using a TA-XT Plus Texture Analyzer equiped with a probe P/0.5R (1/2" diameter Delrin cylinder). The sample is prepared by mixing the ingredients, coating them on the appropriate substrate and curing the coating. Test parameters: test speed =1.0 mm/s; force applied = 100 g; hold time = 5.0 s; trigger force = 1.0 g.
- The peel adhesion in N (newton) is measured using a TA-XT Plus Texture Analyzer or Tensile Testing equipment (e.g. Instron). The sample is prepared by mixing the ingredients, coating them on the appropriate substrate and curing the coating. The coated substrate is then cut in 2.5 cm strips and a polyester film (Mylar) is laminated to the surface. Test parameters: 180° peel release test, test speed = 10.0 mm/s, distance = 110.0 mm.
- The polyurethane adhesion in N (newton) is measured with a TA-XT Plus Texture Analyzer or Tensile Testing equipment (e.g. Instron). The sample is prepared by coating a silicone PSA (Dow Corning® 7-4602) on polyester (Mylar) using a table top coater using shim size = 3 mil. The PSA on Mylar is covered with a suitable release liner, cut into strips (strip size = 2.5 cm wide X 17.5 cm long). Adhesive gel is deposited on polyurethane sheets by mixing the ingredients, coating them on the polyurethane using table top coater using shim size = 10 mil, curing the coating and covering with polyethylene to protect the gel. The sheets are then cut into strips (strip size = 2.5 cm wide X 12.5 cm long) To test, the gel strip is laminated to the PSA strip minimizing air entrapment and a roller is used to adhere samples together. The laminated strips are condition in an oven for 20 minutes at 50°C, allowed to cool completely (minimum 10 minutes), and then test on Texture Analyzer using the gel laminate adhesion test (180° peel test). Each sample was run in triplicate and the average force (N) was reported. Any observed characteristics were noted.

### Materials Used

- vinyl terminated PDMS (polydimthylsiloxane)- 400 mPa.s
   ○ dimethyl siloxane, dimethylvinyl siloxy-terminated
   ○ 0.40 w./w.% vinyl
   ○ Viscosity = 400 mPa.s
- vinyl terminated PDMS - 2,100 mPa.s
   ○ dimethyl siloxane, dimethylvinyl siloxy-terminated
   ○ 0.23 w./w.% vinyl
   ○ Viscosity = 2,100 mPa.s
- vinyl terminated PDMS - 9,500 mPa.s
   ○ dimethyl siloxane, dimethylvinyl siloxy-terminated
   ○ 0.17 w./w.% vinyl
   ○ Viscosity = 9,500 mPa.s
- vinyl substituted MQ resin
   ○ dimethylvinylated and trimethylated silica
   ○ 1.5 - 2.0 w./w.% vinyl
   ○ 1.0 - 1.5 w./w.% OH as SiOH
   ○ Solid
- MQ resin
   ○ silicic acid, sodium salt, reaction products with chlorotrimethyl silane and isopropyl alcohol
   ○ 0 w./w.% vinyl
   ○ 2.0 - 2.5 w./w.% OH as SiOH
   ○ solid
- platinum catalyst
   ○ 1,3-diethenyl-1,1,3,3 -tetramethyldisiloxane platinum complex
   ○ 0.5 w./w.% platinum
   ○ 2.3 w./w.% vinyl
   ○ Viscosity = 400 mPa.s
- Hydrogen-terminated PDMS
   ○ dimethyl siloxane, hydrogen-terminated
   ○ 0.15 w./w.% H as SiH
   ○ Viscosity = 15 mPa.s
- Hydrogen substituted PDMS
   ○ Dimethyl, Methylhydrogen Siloxane, Trimethylsiloxy-terminated
   ○ 0.75 w./w% H as SiH
   ○ Viscosity = 5 mPa.s
- PDMS 100,000 mPa.s
   ○ Dimethyl Siloxane, Trimethylsiloxy-terminated
   ○ Viscosity = 100,000 mPa.s
- Titanate
   ○ Tetrabutoxy Titanate
- UDA (undecylenyl alcohol)
   ○ Undec-10-en-1-ol

### Examples 1-17

### Example 1 (control) -

The following ingredients were mixed in the amounts indicated:

| | |
|---|---|
| vinyl terminated PDMS - 400 mPa.s | 100 parts |
| platinum catalyst | 0.01 % |
| Hydrogen-terminated PDMS | 7 parts |
| Hydrogen substituted PDMS | 0.25% |

The materials were tested with the following results:

| | |
|---|---|
| Viscosity (mPa.s) | 400 |
| Softness (penetration mm/10) | 90 |
| Tack (gf) | 120 |
| Peel adhesion (N) | 0.196 |
| PU adhesion (N) | 1.104 - delaminating from PU surface |

This example shows that gels without the hydroxy substituted siloxane resin (currently proposed for wound dressing application.) had low adhesion to PU.

### Example 2 - (Control)

The following ingredients were mixed in the amounts indicated:

| | |
|---|---|
| vinyl terminated PDMS - 400 mPa.s | 100 parts |
| PDMS 100,000 mPa.s | 8 parts |
| platinum catalyst | 0.01% |
| Hydrogen-terminated PDMS | 7 parts |
| Hydrogen substituted PDMS | 0.25% |

The materials were tested with the following results:

| | |
|---|---|
| Viscosity (mPa.s) | 1200 |
| Softness (penetration mm/10) | 99 |
| Tack(gf) | 137 |
| Peel adhesion (N) | 0.297 |
| PU adhesion (N) | 1.218 - delaminating from PU surface |

This example shows that gels without the hydroxy substituted siloxane resin (but including a skin adhesion enhance) had low adhesion to PU.

### Example 3 - (Control)

The following ingredients were mixed in the amounts indicated:

| | |
|---|---|
| vinyl terminated PDMS - 9,500 mPa.s | 100 parts |
| vinyl terminated PDMS - 2,100 mPa.s | 34 parts |
| platinum catalyst | 0.01 % |
| Hydrogen substituted PDMS | 0.3% |

The materials were tested with the following results:

| | |
|---|---|
| Viscosity (mPa.s) | 7000 |
| Softness (penetration mm/10) | 191 |
| Tack (gf) | 89 |
| Peel adhesion (N) | 0.171 |
| PU adhesion (N) | 0.540 - delaminating from PU surface |

This example shows that gels without the hydroxy substituted siloxane resin (but including a higher Mw vinyl terminated PDMS) had low adhesion to PU.

### Example 4 - (Control)

The following ingredients were mixed in the amounts indicated:

| | |
|---|---|
| vinyl terminated PDMS - 400 mPa.s | 100 parts |
| vinyl substituted MQ resin | 44 parts |
| platinum catalyst | 0.01 % |
| Hydrogen-terminated PDMS | 18 parts |

The materials were tested with the following results:

| | |
|---|---|
| Viscosity (mPa.s) | 1200 |
| Softness (penetration mm/10) | 135 |
| Tack (gf) | 51 |
| Peel adhesion (N) | 0.306 |
| PU adhesion (N) | 1.162 - delaminating from PU surface |

This example shows that gels without the hydroxy substituted siloxane resin (but including a lower amount of a vinyl resin amount) had low adhesion to PU.

### Example 5 - (Control)

The following ingredients were mixed in the amounts indicated: vinyl terminated PDMS - 400 mPa.s 100 parts

| | |
|---|---|
| vinyl substituted MQ resin | 100 parts |
| platinum catalyst | 0.01% |
| Hydrogen-terminated PDMS | 25 parts |
| Hydrogen substituted PDMS | 0.15% |

The materials were tested with the following results:

| | |
|---|---|
| Viscosity (mPa.s) | 5000 |
| Softness (penetration mm/10) | 139 |
| Tack (gf) | 162 |
| Peel adhesion (N) | 0.733 |
| PU adhesion (N) | 1.982 - delaminating from PU surface |

This example shows that gels without the hydroxy substituted siloxane resin (but including a higher amount of a vinyl resin amount) helps to increase the peel adhesion to PU but does not prevent delamination

### Example 6 - (Control)

The following ingredients were mixed in the amounts indicated:

| | |
|---|---|
| vinyl terminated PDMS - 400 mPa.s | 100 parts |
| vinyl substituted resin | 12 parts |
| platinum catalyst | 0.01% |
| Adhesion promoter (titanate) | 1% |
| Hydrogen-terminated PDMS | 14 parts |

The materials were tested with the following results:

| | |
|---|---|
| Viscosity (mPa.s) | 500 |
| Softness (penetration mm/10) | 149 |
| Tack (gf) | 104 |
| Peel adhesion (N) | 0.419 |
| PU adhesion (N) | 2.018 - cohesive failure |

This example shows that gels without the hydroxy substituted siloxane resin (but including a titanate adhesion promoter) have good adhesion to PU

### Example 7 - (Control)

The following ingredients were mixed in the amounts indicated: ditto 6 with UDA

| | |
|---|---|
| vinyl terminated PDMS - 400 mPa.s | 100 parts |
| vinyl substituted MQ resin | 11 parts |
| platinum catalyst | 0.01% |
| Adhesion promoter (UDA) | 2% |
| Hydrogen-terminated PDMS | 10 parts |
| Hydrogen substituted PDMS | 1% |

The materials were tested with the following results:

| | |
|---|---|
| Viscosity (mPa.s) | 500 |
| Softness (penetration mm/10) | 124 |
| Tack (gf) | 29 |
| Peel adhesion (N) | 0.114 |
| PU adhesion (N) | 3.076 - cohesive failure |

This example shows that gels without the hydroxy substituted siloxane resin (but including a UNDECYLENYL ALCOHOL adhesion promoter) have good adhesion to PU

### Example 8 - (Control)

The following ingredients were mixed in the amounts indicated:

| | |
|---|---|
| vinyl terminated PDMS - 400 mPa.s | 100 parts |
| vinyl substituted MQ resin | 11 parts |
| platinum catalyst | 0.01% |
| Adhesion promoter (titanate) | 1% |
| Hydrogen-terminated PDMS | 14 parts |

The materials were tested with the following results:

| | |
|---|---|
| Viscosity (mPa.s) | 450 |
| Softness (penetration mm/10) | 148 |
| Tack (gf) | 56 |
| Peel adhesion (N) | not tested |
| PU adhesion (N) | 1.746 - delaminating from PU surface |

This example shows that gels without the hydroxy substituted siloxane resin (but including a titanate adhesion promoter) have good adhesion to PU

### Example 9 -

The following ingredients were mixed in the amounts indicated:

| | |
|---|---|
| vinyl terminated PDMS - 400 mPa.s | 100 parts |
| vinyl terminated PDMS - 2,100 mPa.s | 22 parts |
| vinyl substituted MQ resin | 25 parts |
| MQ resin | 19 parts |
| platinum catalyst | 0.01% |
| Hydrogen-terminated PDMS | 17 parts |

The materials were tested with the following results:

| | |
|---|---|
| Viscosity (mPa.s) | 750 |
| Softness (penetration mm/10) | 87 |
| Tack (gf) | 132 |
| Peel adhesion (N) | 0.717 |
| PU adhesion (N) | 4.978 - delaminating from the test substrate |

This example shows that gels with the hydroxy substituted siloxane resin had good adhesion to PU.

### Example 10 - (replicate of example 9)

The following ingredients were mixed in the amounts indicated:

| | |
|---|---|
| vinyl terminated PDMS - 400 mPa.s | 100 parts |
| vinyl terminated PDMS - 2,100 mPa.s | 22 parts |
| vinyl substituted MQ resin | 25 parts |
| MQ resin | 19 parts |
| platinum catalyst | 0.01% |
| Hydrogen-terminated PDMS | 17 parts |

The materials were tested with the following results:

| | |
|---|---|
| Viscosity (mPa.s) | 900 |
| Softness (penetration mm/10) | 102 |
| Tack (gf) | 142 |
| Peel adhesion (N) | 0.737 |
| PU adhesion (N) | 4.451 - delaminating from the test substrate |

This example shows that gels with the hydroxy substituted siloxane resin had good adhesion to PU.

### Example 11 -

The following ingredients were mixed in the amounts indicated:

| | |
|---|---|
| vinyl terminated PDMS - 400 mPa.s | 100 parts |
| vinyl terminated PDMS - 2,100 mPa.s | 18 parts |
| vinyl substituted PDMS | 2 parts |
| vinyl substituted MQ resin | 9 parts |
| MQ resin | 20 parts |
| platinum catalyst | 0.01% |
| Hydrogen-terminated PDMS | 18 parts |

The materials were tested with the following results:

| | |
|---|---|
| Viscosity (mPa.s) | 800 |
| Softness (penetration mm/10) | 96 |
| Tack (gf) | 131 |
| Peel adhesion (N) | 0.283 |
| PU adhesion (N) | 1.977 - delaminating from the test substrate |

This example shows that gels with the hydroxy substituted siloxane resin had good adhesion to PU.

### Example 12 -

The following ingredients were mixed in the amounts indicated:

| | |
|---|---|
| vinyl terminated PDMS - 400 mPa.s | 100 parts |
| vinyl terminated PDMS - 2,100 mPa.s | 16 parts |
| vinyl substituted MQ resin | 18 parts |
| MQ resin | 20 parts |
| platinum catalyst | 0.01% |
| Hydrogen-terminated PDMS | 20 parts |

The materials were tested with the following results:

| | |
|---|---|
| Viscosity (mPa.s) | 750 |
| Softness (penetration mm/10) | 86 |
| Tack (gf) | 79 |
| Peel adhesion (N) | 0.105 |
| PU adhesion (N) | 1.170 - delaminating from the test substrate |

This example shows that gels with the hydroxy substituted siloxane resin had good adhesion to PU.

## Claims

1. A silicone gel containing between 2 and 45 wt. % of at least one hydroxy substituted siloxane resin comprising R₃SiO_{1/2} units and SiO_{4/2} units wherein each R is independently a linear, branched or cyclic hydrocarbon group having 1 to 20 carbon atoms and wherein the molar ratio of R₃SiO_{1/2} to SiO_{4/2} is from 0.6:1 to 4:1, for use in adhering a medical substrate to a human or animal comprising affixing a layer of the silicone gel between a medical substrate and a human or animal.

2. A silicone gel for use in adhering a medical substrate to a human or animal according to claim 1 in which the silicone gel is the reaction product of a gel forming composition comprising:
10% to 90 wt.% of at least one alkenyl-substituted polydiorganosiloxane;
2% to 45 wt% of at least one hydroxy functional siloxane resin;
1% to 30% wt. of at least one organosiloxane containing silicon-bonded hydrogen atoms; and
at least one hydrosilylation catalyst.

3. The silicone gel for use in adhering a medical substrate to a human or animal according to claim 1 in which the medical substrate is made of polyurethane.

4. The silicone gel for use in adhering a medical substrate to a human or animal according to Claim 2 in which the gel forming composition also comprises an active agent.

5. A silicone gel comprising the reaction product of a composition comprising:
10% to 90 wt.% of at least one alkenyl-substituted polydiorganosiloxane;
2% to 45 wt.% of at least one hydroxy functional siloxane resin comprising R₃SiO_{1/2} units and SiO_{4/2} units wherein each R is independently a linear, branched or cyclic hydrocarbon group having 1 to 20 carbon atoms and wherein the molar ratio of R₃SiO_{1/2} to SiO_{4/2} is from 0.6:1 to 4:1;
1% to 30% wt. of at least one organosiloxane containing silicon-bonded hydrogen atoms; and
at least one hydrosilylation catalyst.

6. The silicone gel according to claim 5 in which the composition also comprises an active agent.

7. The silicone gel according to claim 5 or 6 in which the composition also comprises a solvent.

8. The silicone gel according to claim 5 or 6 in which the composition is solventless.

## Patentansprüche

1. Silicongel, enthaltend zwischen 2 und 45 Gew.-% mindestens eines hydroxysubstituierten Siloxanharzes, das R₃SiO_{1/2}-Einheiten und SiO_{4/2}-Einheiten enthält, wobei jedes R unabhängig voneinander eine lineare, verzweigte oder cyclische Kohlenwasserstoffgruppe mit 1 bis 20 Kohlenstoffatomen ist und wobei das Molverhältnis von R₃SiO_{1/2}:SiO_{4/2} von 0,6:1 bis 4:1 reicht, zur Verwendung beim Anhaften eines medizinischen Substrats an einen Mensch oder ein Tier, umfassend Anbringen einer Schicht des Silicongels zwischen einem medizinischen Substrat und einem Menschen oder Tier.

2. Silicongel zur Verwendung bei der Anhaftung eines medizinischen Substrats an einen Menschen oder ein Tier gemäß Anspruch 1, wobei das Silicongel das Reaktionsprodukt einer gelbildenden Zusammensetzung ist, die enthält:
10 bis 90 Gew.-% mindestens eines alkenylsubstituierten Polydiorganosiloxans;
2 bis 45 Gew.-% mindestens eines hydroxyfunktionellen Siloxanharzes;
1 bis 30 Gew.-% mindestens eines Organosiloxans, das siliciumgebundene Wasserstoffatome enthält, und
mindestens einen Hydrosilylierungskatalysator.

3. Silicongel zur Verwendung bei der Anhaftung eines medizinischen Substrats an einen Menschen oder ein Tier gemäß Anspruch 1, in welchem das medizinische Substrat aus Polyurethan hergestellt ist.

4. Silicongel zur Verwendung bei der Anhaftung eines medizinischen Substrats an einen Menschen oder ein Tier gemäß Anspruch 2, in welchem die gelbildende Zusammensetzung ferner ein aktives Mittel enthält.

5. Silicongel, enthaltend das Reaktionsprodukt einer Zusammensetzung, die enthält:
10 bis 90 Gew.-% mindestens eines alkenylsubstituierten Polydiorganosiloxans;
2 bis 45 Gew.% mindestens eines hydroxyfuriktionellen Siloxanharzes, enthaltend R₃SiO_{1/2}-Einheiten und SiO_{4/2}-Einheiten, wobei jedes R unabhängig voneinander eine lineare, verzweigte oder cyclische Kohlenwasserstoffgruppe mit 1 bis 20 Kohlenstoffatomen ist und wobei das Molverhältnis von R₃SiO_{1/2}:SiO_{4/2} von 0,6:1 bis 4:1 reicht;
1 bis 30 Gew.-% mindestens eines Organosiloxans, das siliciumgebundene Wasserstoffatome enthält, und
mindestens einen Hydrosilyllerungskatalysator.

6. Silicongel gemäß Anspruch 5, in welchem die Zusammensetzung ferner ein aktives Mittel enthält.

7. Silicongel gemäß Anspruch 5 oder 6, in welchem die Zusammensetzung ferner ein Lösungsmittel enthält.

8. Silicongel gemäß Anspruch 5 oder 6, in welchem die Zusammensetzung lösungsmittelfrei ist.

## Revendications

1. Gel de silicone contenant une quantité comprise entre 2 et 45 % en poids d'au moins une résine de siloxane substituée par un groupement hydroxyle comprenant des unités R₃SiO_{1/2} et des unités SiO_{4/2} dans laquelle chaque radical R représenté indépendamment un groupement hydrocarboné linéaire, ramifié ou cyclique ayant 1 à 20 atomes de carbone et dans laquelle le rapport molaire des unités R₃SiO_{1/2} aux unités SiO_{4/2} est de 0,6:1 à 4:1, pour une utilisation destinée à faire adhérer un substrat médical sur un être humain ou sur un animal comprenant la fixation d'une couche du gel de silicone entre un substrat médical et un être humain ou un animal.

2. Gel de silicone pour une utilisation destinée à faire adhérer un substrat médical sur un être humain ou sur un animal selon la revendication 1, dans lequel le gel de silicone est le produit réactionnel d'une composition filmogène comprenant :
- 10 % à 90 % en poids d'au moins un polydiorganosiloxane substitué par un alcényle ;
- 2 % à 45 % en poids d'au moins une résine de siloxane à fonction hydroxyle ;
- 1 % à 30 % en poids d'au moins un organosiloxane contenant des atomes d'hydrogène liés à un atome de silicium ; et
au moins un catalyseur d'hydrosilylation.

3. Gel de silicone pour une utilisation destinée à faire adhérer un substrat médical sur un être humain ou sur un animal selon la revendication 1, dans lequel le substrat médical est fabriqué en polyuréthane.

4. Gel de silicone pour une utilisation destinée à faire adhérer un substrat médical sur un être humain ou sur un animal selon la revendication 2, dans lequel la composition filmogène comprend également un agent actif.

5. Gel de silicone comprenant le produit réactionnel d'un composition comprenant :
- 10 % à 90 % en poids d'au moins un polydiorganosiloxane substitué par un alcényle ;
- 2 % à 45 % en poids d'au moins une résine de siloxane à fonction hydroxyle comprenant des unités R₃SiO_{1/2} et des unités SiO_{4/2} dans laquelle chaque radical R représente indépendamment un groupement hydrocarboné linéaire, ramifié ou cyclique ayant 1 à 20 atomes de carbone et dans laquelle le rapport molaire des unités R₃SiO_{1/2} aux unités SiO_{4/2} est de 0,6:1 à 4:1 ;
- 1 % à 30 % en poids d'au moins un organosiloxane contenant des atomes d'hydrogène liés à un atome de silicium ; et
au moins un catalyseur d'hydrosilylation.

6. Gel de silicone selon la revendication 5, dans lequel la composition comprend également un agent actif.

7. Gel de silicone selon la revendication 5 ou 6, dans lequel la composition comprend également un solvant.

8. Gel de silicone selon la revendication 5 ou 6, dans lequel la composition ne comprend pas de solvant.
